(19) Europäisches Patentamt
European Patent Office
Office européen des brevets

(11) EP 2 616 808 B1

(12) # EUROPEAN PATENT SPECIFICATION

(45) Date of publication and mention of the grant of the patent:
**25.02.2015 Bulletin 2015/09**

(21) Application number: **11825746.8**

(22) Date of filing: **12.09.2011**

(51) Int Cl.:
***G01N 33/53*** *(2006.01)* ***G01N 33/573*** *(2006.01)*
***G01N 33/52*** *(2006.01)* ***C12Q 1/37*** *(2006.01)*

(86) International application number:
**PCT/US2011/051274**

(87) International publication number:
**WO 2012/037044 (22.03.2012 Gazette 2012/12)**

(54) **PROLYL ENDOPEPTIDASE PROBES**

PROLYLENDOPEPTIDASESONDEN

SONDES DE LA PROLYLE ENDOPEPTIDASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.09.2010 US 382315 P**

(43) Date of publication of application:
**24.07.2013 Bulletin 2013/30**

(73) Proprietor: **SRI International**
**Menlo Park, CA 94025-3493 (US)**

(72) Inventors:
- **GALANDE, Amit, K.**
**Harrisonburg**
**VA 22802 (US)**
- **WATSON, Douglas, Stuart**
**Harrisonburg**
**VA 22802 (US)**
- **KODUKULA, Krishna**
**Harrisonburg**
**VA 22802 (US)**
- **JAMBUNATHAN, Kalyani**
**Harrisonburg**
**VA 22802 (US)**

(74) Representative: **Viering, Jentschura & Partner**
**Patent- und Rechtsanwälte**
**Kennedydamm 55 / Roßstrasse**
**40476 Düsseldorf (DE)**

(56) References cited:
**WO-A2-2007/087131 US-A1- 2003 096 328**
**US-A1- 2007 207 499**

- **ORLOWSKI M ET AL: "PROTEOLYTIC ENZYMES IN BRONCHO PULMONARY LAVAGE FLUIDS CATHEPSIN B-LIKE ACTIVITY AND PROLYL ENDO PEPTIDASE", JOURNAL OF LABORATORY AND CLINICAL MEDICINE, vol. 97, no. 4, 1981, pages 467-476, XP008164470, ISSN: 0022-2143**
- **QUINTO B M R ET AL: "Characterization of a prolyl endopeptidase (kininase) from human urine using fluorogenic quenched substrates", INTERNATIONAL JOURNAL OF BIOCHEMISTRY AND CELL BIOLOGY, vol. 32, no. 11-12, November 2000 (2000-11), pages 1161-1172, XP055076824, ISSN: 1357-2725**
- **FULOP VILMOS ET AL: "Structures of prolyl oligopeptidase substrate/inhibitor complexes: Use of inhibitor binding for titration of the catalytic histidine residue", JOURNAL OF BIOLOGICAL CHEMISTRY, vol. 276, no. 2, 12 January 2001 (2001-01-12), pages 1262-1266, XP055076825, ISSN: 0021-9258**
- **FULOP, V. ET AL.: 'Structures of prolyl oligopeptidase substrate/inhibitor complexes. Use of inhibitor binding for titration of the catalytic histidine residue.' J. BIOL. CHEM. vol. 276, 2001, pages 1262 - 1266**
- **VENDEVILLE, S. ET AL.: 'Comparison of the inhibition of human and Trypanosoma cruzi prolyl endopeptidases.' BIOORGANIC & MEDICINAL CHEMISTRY vol. 10, 2002, pages 1719 - 1729**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 616 808 B1

- CUNNINGHAM, D. F. ET AL.: 'Identification and initial characterisation of a N-benzyloxycarbonyl-prolyl-prolinal(Z-Pro-p rolinal)-insensitive 7-(N-benzyloxycarbonyl-glycyl-prolyl-amido)-4-me thylcoumarin)Z-Gly- Pro-NH-Mec)-hydrolysing peptidase in bovine serum.' EUR. J. BIOCHEM. vol. 244, 1997, pages 900 - 903

**Description**

FIELD OF THE INVENTION

The field of the invention are methods of detecting prolyl endopeptidase activity.

BACKGROUND OF THE INVENTION

Poor diagnosis is a major problem when deciding treatment options for patients suffering from infectious diseases. Sensitive and specific assays for the reliable detection of existing and emerging pathogens are needed.

The invention involves using a peptide based probe which when added to a biological sample of an infected patient, results in signal generation (e.g. fluorescence). This invention enables identification and preparation of specific peptide-based fluorogenic probes that can be used in a fluorescence based assay to detect signature proteolytic activity in the biological fluids of human hosts. The inventors have established proteolytic activity as a biomarker for rapid and accurate assessment of individuals' health status.

The invention provide an entirely new class of in vitro diagnostics to detect a variety of infectious diseases with high sensitivity and specificity. Health care providers can effectively use this invention as a part of their diagnostics platform. The probes can also be used to monitor therapy, wherein early diagnosis and treatment could cut mortality in half for many infectious diseases.

Orlowski et al. (Orlowski. M. et al. (1981) Journal of laboratory and clinical medicine, 97, 4, 467-476) disclose prolyl endopeptidase activity in bronchopulmonary lavage fluids. Quinto et al. (Quinto, B.M.R. et al. (2000), International Journal of Biochemistry and Cell Biology, 32, 11-12, 1161-1172), Fulop Vilmos et al. (Fulop Vilmos et al. (2001), Journal of Biological Chemistry, 276, 2, 1262-1266) and WO 2007/087131 A2 disclose the detection of prolyl endopeptidase activity.

Aspects of this disclosure were published by the inventors in: Watson et al., BioTechiques 51, 95-104 (August 2011) and Watson et al., PLoS ONE June 2011, 6, 6, e21001.

SUMMARY OF THE INVENTION

The invention is defined by the appended claims.

The invention provides methods of detecting prolyl endopeptidase (PE) activity in a lung sample.

The method comprises the steps of: (a) contacting the sample with a probe comprising a -P-X- (or -X-P- or -P-X-P-) PE recognition site, wherein P is a prolyl bioisostere, X is (i) N, F, Y, S, H or G, or (ii) a prolyl bioisostere flanked on each side by N, F, Y, S, H or G, or (iii) aminoluciferin, and "-" is an amide bond, under conditions wherein PE activity of the sample specifically hydrolyzes an amide bond of the recognition site to generate an optical signal; and (b) detecting the signal.

In particular embodiments:

the probe comprises an -P-X-P-X- or P-X-P-X-P- recognition site, particularly wherein X is a residue that is not a prolyl bioisostere;

the prolyl bioisostere is selected from proline, dehydroproline, homoproline, N-methylamino acid, and decahydroisoquinoline carboxylate, each of which may be optionally substituted;

the probe further comprises a hydrophilic moiety and the probe is water soluble; the probe is internally quenched;

one end of the recognition site is operably-linked to a first chromophore (such as a FRET donor or a fluorophore), and the other end of the recognition site is operably-linked to a second, different chromophore (such as a FRET acceptor or a quenching moiety), particularly wherein the chromophores are independently operably-linked to the recognition site through a linker that is glycine, serine, a peptide of serine and/or glycine, a mini-PEG (8-amino-3,6-dioxaoctanoic acid or 11-amino-3,6,9-trioxaundecanoic acid) or a linear aliphatic alpha-amino acid;

proteolysis of the probe produces a substrate, and the sample is further contacted with an enzyme that reacts with the substrate to produce the signal;

the sample is bronchoalveolar lavage fluid of a patient having a lung disease, particularly a lung infection that is a fungal infection, a bacterial infection, a parasitic infection, or a viral infection;

the lung has a disease that is a non-infectious inflammatory disease, including chronic or acute inflammatory disease; and/or

the contacting and detecting steps are repeated with different PE probes, particularly with different lung samples, wherein the method determines one or more differences in PE activity between the samples.

Described herein are also methods of detecting prolyl endopeptidase (PE) activity in each of a plurality of

samples. The method comprises the steps of: (a) contacting each sample with a panel of different probes each comprising a -P-X- (or -P-X-P-) PE recognition site, wherein P is a prolyl bioisostere, X is a residue that is not a prolyl bioisostere or is a prolyl bioisostere flanked on each side by a residue that is not a prolyl bioisostere, and "-" is an amide bond, under conditions wherein PE activity of the sample specifically hydrolyzes an amide bond of the recognition site to generate an optical signal; and (b) detecting and comparing the resultant signals from each sample to determine differences in PE activity between the samples.

Described herein are probes adapted to the subject methods. Further herein described is an internally quenched fluorogenic probe (IQFP) for prolyl endopeptidase (PE) activity comprising a -P-X (or -P-X-P-) PE recognition site, wherein P is a prolyl bioisostere, X is a residue that is not a prolyl bioisostere or is a prolyl bioisostere flanked on each side by a residue that is not a prolyl bioisostere, and "-" is an amide bond, wherein one end of the recognition site is operably-linked to a FRET donor, and the other end of the recognition site is operably-linked to a FRET acceptor, wherein PE hydrolysis of an amide bond of the recognition generates an optical signal.

The donor and acceptor may be independently operably-linked through a linker that is glycine, serine, a peptide of serine and/or glycine, a mini-PEG (8-amino-3,6-dioxaoctanoic acid or 11-amino-3,6,9-trioxaundecanoic acid) or a linear aliphatic alpha-amino acid.

Further described herein are methods of making and using the probes in the disclosed methods, including diagnostic, characterization and screening methods.

The invention provides all combinations and subcombinations of recited particular embodiments as if each combination and subcombination had been specifically, separately recited.

BRIEF DESCRIPTION OF THE FIGURES

FIG. 1. General structure of fluorogenic probes for detection of invasive aspergillosis.
FIG. 2. BALF IQFP assay: PNP
FIG. 3. BALF IQFP assay: PYP
FIG. 4. BALF assay: PYP
FIG. 5. BALF v. Serum
FIG. 6. Infected v. Uninfected
FIG. 7. (A) Amino acid distribution of sequences cleaved by BALF; (B) Amino acid distribution of sequences cleaved by serum; (C) Motifs cleaved by BALF; (D) Motifs cleaved by serum; (E) Distribution of BALF protease cleavage sites; (F) Distribution of BALF serum cleavage sites.

DETAILED DESCRIPTIO OF SPECIFIC EMBODIMENTS OF THE INVENTION

Described herein is a peptide based probe comprising a protease substrate that has a specific sequence and consists of 3 to 10 amino acids. On either side of this peptide sequence is located a chromophore and/or a fluorophore. This probe remains optically silent when added to a biological fluid of a healthy patient, but when added to a biological fluid of an infected patient it emits a fluorescence signal, typically with emission the range of 300 nm to 800 nm. Assayable biological fluids include serum and bronchoalveolar lavage fluid, as well as urine, blood, plasma, and saliva. Lung-derived samples may be obtained from biopsy samples, BALF, etc. The probes can be used for the detection of fungal diseases, such as invasive aspergillosis, as well as bacterial diseases (e.g. tuberculosis, MRSA), parasitic diseases (e.g. Leishmaniasis, Chagas disease), and viral diseases (e.g. H1N1, SARS).

The invention is generally applicable in the in vitro diagnostics industry. Specifically, a panel of multiple fluorogenic probes can be used to detect a variety of pathogenic conditions in parallel. The probes can also be used for detecting the level of infection (e.g. invasion vs. localization), response to antimicrobials (e.g. recovery), and to diagnose the general health status of individuals.

In a particular embodiment the invention provides detection of protease activity by luminescence, for the diagnosis of infection or other uses, e.g. using a peptide based probe which when added to a biological sample of an infected patient, followed by sequential addition of a recombinant enzyme (such as luciferase) results in generation of luminescence.

The peptide based probe consists of a protease substrate that has a specific sequence and consists of 3 to 10 amino acids. The probe contains a luminescent substrate, such as aminoluciferin, either within the substrate sequence or in flanking regions at the N- or C-terminus. Cleavage of the substrate by one or more proteases results in liberation of the luminescent substrate, which is chemically modified by a recombinant enzyme, resulting in the generation of light. This probe remains optically silent when added to a biological fluid of a healthy patient, but when added to a biological fluid of an infected patient it emits a luminescent signal with emission anywhere in the range of 300 nm to 800 nm. The

probes are useful for the detection of fungal diseases, such as invasive aspergillosis, as well as bacterial diseases (e.g. tuberculosis, MRSA), parasitic diseases (e.g. Leishmaniasis, Chagas disease), and viral diseases (e.g. H1N1, SARS).

A specific example of this aspect is the probe sequence GGGPAlucPGGKK, where Aluc corresponds to aminoluciferin and all other letters correspond to natural amino acids according to the single letter code. Cleavage of this sequence by an endopeptidase generates the peptide fragment AlucPGGKK. Subsequent cleavage by an protease liberates the Aluc moiety, which is converted to aminooxyluciferin by the addition of luciferase enzyme, resulting in the generation of light.

In a particular embodiment the disclosure provides imaging pulmonary infection or inflammation with protease substrate probes, e.g. using imaging techniques to identify specific areas of infection or inflammation within the lungs in vivo to facilitate targeted therapy or surgery. The disclosure provides an imaging agent comprising a protease substrate sequence attached to a reporter moiety. The substrate sequence corresponds to a protease that is present during a specific infection, such as invasive aspergillosis, or an inflammatory condition, such as cystic fibrosis. The imaging agent is administered by the pulmonary or intravenous route. Cleavage of the substrate sequence in the presence of a specific protease results in activation of the attached reporter. Examples of reporter modalities include fluorescence, luminescence, PET, MRI, and SPECT, among others. This aspect is useful for determination of specific localized sites of infection of inflammation within the lung for many diseases, including pneumonia, bronchiectasis, emphysema, tuberculosis, fibrosis, chronic obstructive pulmonary disease, and asthma.

In a particular embodiment the disclosure provides peptides of the sequence X-P-X-P-X as biomarkers for infection or inflammation. The inventors have found that proteases that generate peptides of the sequence proline-glycine-proline are elevated in pulmonary inflammatory conditions, such as invasive aspergillosis and cystic fibrosis. The presence of short peptide fragments produced by these proteases provide useful biomarkers for the presence or extent of disease. Useful peptides include sequences that include X-P-X-P-X, where P corresponds to proline and X corresponds to any sequence as described in the appended claims.

In one aspect the invention provides a method of detecting prolyl endopeptidase (PE) activity in a lung sample, comprising the steps of: (a) contacting the sample with a probe comprising a -P-X- (or -X-P-or -P-X-P-) PE recognition site, wherein P is a prolyl bioisostere, X is (i) N, F, Y, S, H or G, or (ii) a prolyl bioisostere flanked on each side by N, F, Y, S, H or G, or (iii) aminoluciferin, and "-" is an amide bond, under conditions wherein PE activity of the sample specifically hydrolyzes an amide bond of the recognition site to generate an optical signal; and (b) detecting the signal.

In particular embodiments the probe comprises a -P-X-, P-X-P-, -P-X-P-X- or -P-X-P-X-Precognition site, wherein X is (i) N, F, Y, S, H or G, or (ii) a prolyl bioisostere flanked on each side by N, F, Y, S, H or G, or (iii) aminoluciferin. Such amino acid alternatives may be used to modulate its stability, solubility or other functionalities. In a particular embodiment the residue contributes to the signal, such as providing a signal generating enzyme substrate, e.g. is aminoluciferin.

Prolyl bioisosteres are well-established structural and functional analogs of proline (e.g. Sampognaro et al., Bioorg Med Chem Lett. 2010 Sep 1;20(17):5027-30), and those used herein are substitutable for proline in peptide probes for prolyl endopeptidase. The prolyl bioisosteres may be generated from alternative scaffolds, such as proline, cyclopentene, cyclohexene, pyrrolidine, pyrrolidinone, carbocyclic, acyclic groups and 4-phenyl-2-carboxy-piperazine (e.g. Nilsson et al., J Comb Chem. 2001 Nov-Dec;3(6):546-53; Thorstensson 2005, Linköping Studies in Science and Technology, Dissertations No. 990). In particular embodiments the prolyl bioisostere is selected from proline, homoproline, hydroxyproline, dehydroproline, aminoproline, 5,6-benzohomoproline (Shuman et al. J Org Chem 1990, 55, 738-41), alkylproline, N-methylamino acid, and decahydroisoquinoline carboxylate, each of which may be optionally substituted with hydroxy, halogen, substituted or unsubstituted alkyl, substituted or unsubstituted alkenyl, substituted or unsubstituted alkynyl, substituted or unsubstituted arylalkyl, substituted or unsubstituted amine, substituted or unsubstituted alkylamine, substituted or unsubstituted dialkylamine, substituted or unsubstituted alkoxy, substituted or unsubstituted aryl, substituted or unsubstituted heteroaryl, substituted or unsubstituted aryoxy, substituted or unsubstituted alkoxy, substituted or unsubstituted haloalkyl, benzo, NO2, CF3, aryl, carboxyl, cyano, isocyanate, or alkoxycarbonyl.

Examples of suitable prolyl bioisosteres include:

| | |
|---|---|
| 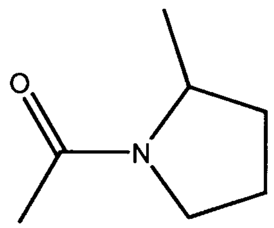 | 1-(2-methylpyrrolidin-1-yl)ethanone |

(continued)

| | |
|---|---|
| 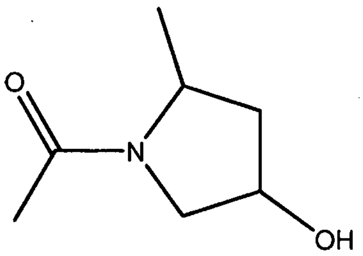 | 1-(4-hydroxy-2-methylpyrrolidin-1-yl)ethanone |
| 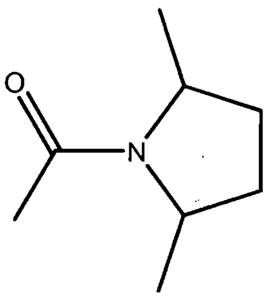 | 1-(2-methylpyrrolidin-1-yl)ethanone |
| 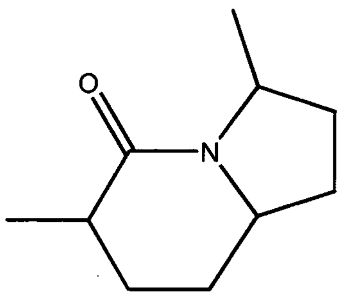 | 3,6-dimethylhexahydroindolizin-5(1*H*)-one |
| 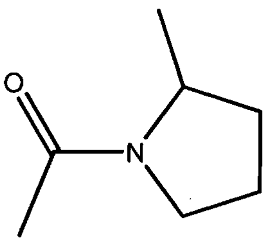 | 1-(optionally-substituted, 2-methylpyrolidin-1-yl)ethanone |
| 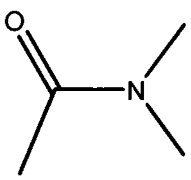 | *N,N*-dimethylacetamide |
| 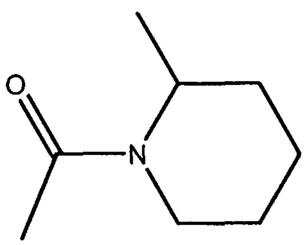 | 1-(2-methylpiperidin-1-yl)ethanone |

(continued)

| | |
|---|---|
| 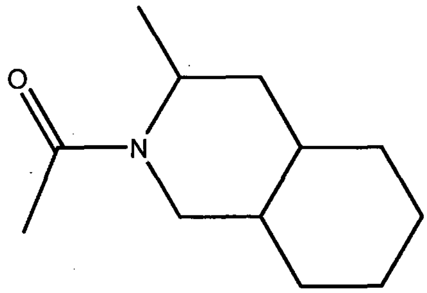 | 1-(3-methyloctahydroisoquinolin-2(1*H*)-yl)ethanone |
| 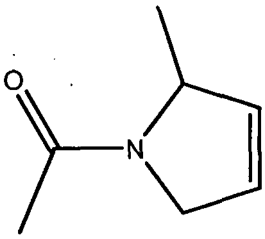 | 1-(2-methyl-2,5-dihydro-1*H*-pyrrol-1-yl)ethanone |
| 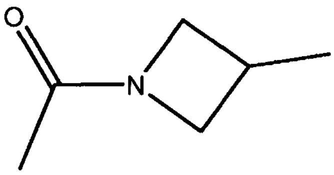 | 1-(3-methylazetidin-1-yl)ethanone |
| 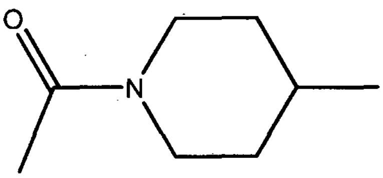 | 1-(4-methylpiperidin-1-yl)ethanone |
| 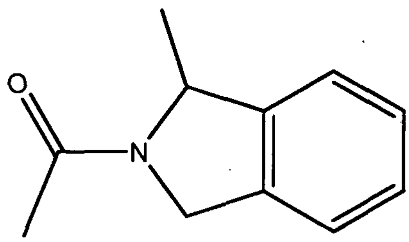 | 1-(1-methylisoindolin-2-yl)ethanone |
| 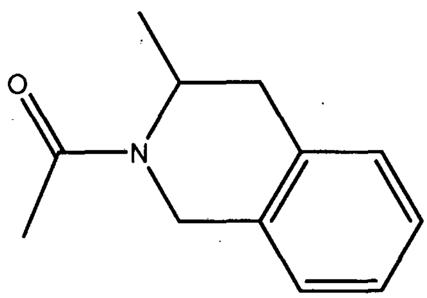 | 1-(3-methyl-3,4-dihydroisoquinolin-2(1*H*)-yl)ethanone |
| 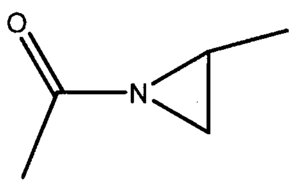 | 1-(2-methylaziridin-1-yl)ethanone |

(continued)

| | |
|---|---|
| 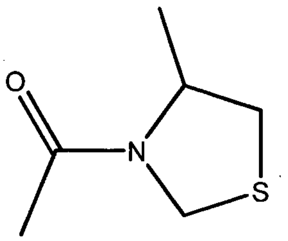 | 1-(4-methylthiazolidin-3-yl)ethanone |
| 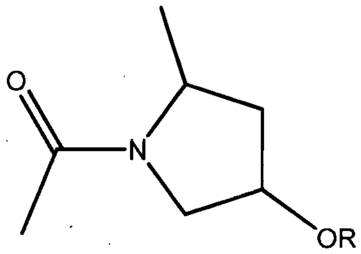 | 1-(2-methyl, 4-R-oxy-1-yl)ethanone |

In particular embodiments the probe further comprises one or more additional flanking sequences to modulate its stability, solubility, or other functionality. In particular embodiments the probe comprises a hydrophilic moiety, such as polylysine (e.g. KKK) to increase water solublility.

In particular embodiments the probe is internally quenched, particularly wherein one end of the recognition site is operably-linked to a fluorescence resonance energy transfer (FRET) donor (or a fluorophore), and the other end of the recognition site is operably-linked to a FRET acceptor (or a quenching moiety). In other embodiments, one end of the recognition site is operably-linked to a first chromophore, and the other end of the recognition site is operably-linked to a second chromophore, wherein fluorescence emission of one chromophore is quenched by the other chromophore, which may be by FRET, aggregation, or other mechanism of contact or non-contact quenching. In particular embodiments, the donor and acceptor (or fluorophore and quenching moiety, or first and second chromophores) are independently operably-linked through a linking residue that is glycine, serine, a peptide of serine and/or glycine, a mini-PEG (8-Amino-3,6-dioxaoctanoic acid or 11-amino-3,6,9-trioxaundecanoic acid) or a linear aliphatic alpha-amino acid (e.g. 6-aminoxexanoic acid). A variety of alternative FRET structures may be incorporated to modulate signaling or other functionality of the probe; for example, in particular embodiments the probe comprises a plurality of donors and/or a plurality of acceptors.

In particular embodiments the sample is bronchoalveolar lavage fluid of a patient having a lung disease, such as a lung infection or lung inflammation, particularly lung inflammation as a results of an acute or chronic health conditions, autoimmune disease or trauma, and particularly in immuo-compromised patients. In particular embodiments the lung and/or patient has a fungal infection (e.g. invasive aspergillosis), a bacterial infection (e.g. tuberculosis, MRSA), a parasitic infection (e.g. leishmaniasis, chagas disease), or a viral infection (e.g. H1N1, SARS). In other embodiments, the lung and/or patient has a disease that is a non-infectious inflammatory disease, including chronic (e.g. chronic obstructive pulmonary disorder, chronic asthma, bronchiectasis, chronic bronchitis, emphysema, pulmonary fibrosis) or acute (trauma, acute asthma, hypersensitivity) inflammatory disease.

The probe signal is detected by convenient means, depending on the selected probe structure including optical signal detection (e.g. colorimetric, luminescent or fluorescence-based assay) mass detection (e.g. mass spectroscopy) or nuclear detection (e.g. NMR, radiography). A variety of assay formats may be employed, including ELISA, RIA, chromatography, microscopy, etc.

The invention also provides methods of using libraries or panels of probes to characterize and/or diagnose disease, particularly wherein the contacting and detecting steps are repeated with different PE probes, particularly with different lung samples, wherein the method determines one or more differences in PE activity between the samples. Further herein described are methods of identifying probes that specifically detect PE activity as biomarkers of a disease condition, particularly in a lung sample, and methods of identifying probes that distinguish between two or more distinct prolyl endopeptidase enzymes.

In particular embodiments, described herein are methods of detecting prolyl endopeptidase (PE) activity in each of a plurality of samples. In one embodiment the method comprises the steps of: (a) contacting each sample with a panel of different probes each comprising a - P-X- (or -P-X-P-) PE recognition site, wherein P is a prolyl bioisostere, X is a residue that is not a prolyl bioisostere or is a prolyl bioisostere flanked on each side by a residue that is not a prolyl bioisostere, and "-" is an amide bond, under conditions wherein PE activity of the sample specifically hydrolyzes an

amide bond of the recognition site to generate an optical signal; and (b) detecting and comparing the resultant signals from each sample to determine differences in PE activity between the samples.

Described herein are probes adapted to the subject methods. Further herein described is an internally quenched fluorogenic probe (IQFP) for prolyl endopeptidase (PE) activity comprising a -P-X (or -P-X-P-) PE recognition site, wherein P is a prolyl bioisostere, X is a residue that is not a prolyl bioisostere or is a prolyl bioisostere flanked on each side by a residue that is not a prolyl bioisostere, and "-" is an amide bond, wherein one end of the recognition site is operably-linked to a FRET donor, and the other end of the recognition site is operably-linked to a FRET acceptor, wherein PE hydrolysis of an amide bond of the recognition generates an optical signal.

In particular embodiments: the donor and acceptor are independently operably-linked through a linking residue that is glycine, polyglycine or a mini-PEG (8-amino-3,6-dioxaoctanoic acid or 11-amino-3,6,9-trioxaundecanoic acid) or a linear aliphatic alpha-amino acid (e.g. 6-aminoxexanoic acid).

Further described herein are methods of making and using the subject probes in the disclosed methods, including diagnostic, characterization and screening methods.

EXAMPLES

Example 1

Specific Examples of Unique Fluorogenic Probes for Detection of Invasive Aspergillosis

Specific examples of unique fluorogenic probes for detection of invasive aspergillosis include the following sequences in the format of "MeOCGly-G-G-X1-X2-X3-G-G-Dap(Dnp)-K-K" where MeOCGly corresponds to 7-methylcoumarin-4-acetyl glycine, Dap(Dnp) corresponds to diaminoproprionyl(dinitrophenyl), and X1-X3 correspond to variable natural amino acids as depicted in FIG. 1. All other letters correspond to natural amino acids according to standard single letter code:

1) MeOCGly-G-G-S/T-I/L-F/Y-G-G-Dap(Dnp)-K-K
2) MeOCGly-G-G-S/T-I/L-N/Q-G-G-Dap(Dnp)-K-K
3) MeOCGly-G-G-I/L-F/Y-F/Y-G-G-Dap(Dnp)-K-K
4) MeOCGly-G-G-A/V-I/L-I/L-G-G-Dap(Dnp)-K-K
5) MeOCGly-G-G-P-A/V-N/Q-G-G-Dap(Dnp)-K-K
6) MeOCGly-G-G-P-A/V-P-G-G-Dap(Dnp)-K-K
7) MeOCGly-G-G-P-K/R-P-G-G-Dap(Dnp)-K-K
8) MeOCGly-G-G-P-D/E-K/R-G-G-Dap(Dnp)-K-K
9) MeOCGly-G-G-P-D/E-P-G-G-Dap(Dnp)-K-K
10) MeOCGly-G-G-P-N/Q-A/V-G-G-Dap(Dnp)-K-K
11) MeOCGly-G-G-P-N/Q-P-G-G-Dap(Dnp)-K-K
12) MeOCGly-G-G-P-I/L-P-G-G-Dap(Dnp)-K-K
13) MeOCGly-G-G-P-S/T-A/V-G-G-Dap(Dnp)-K-K
14) MeOCGly-G-G-P-S/T-P-G-G-Dap(Dnp)-K-K
15) MeOCGly-G-G-P-S/T-F/Y-G-G-Dap(Dnp)-K-K
16) MeOCGly-G-G-P-F/Y-A/V-G-G-Dap(Dnp)-K-K
17) MeOCGly-G-G-P-F/Y-K/R-G-G-Dap(Dnp)-K-K
18) MeOCGly-G-G-P-F/Y-N/Q-G-G-Dap(Dnp)-K-K
19) MeOCGly-G-G-P-F/Y-P-G-G-Dap(Dnp)-K-K

Additional specific examples of unique fluorogenic probes for detection of invasive aspergillosis include the following sequences in the format of "MeOCGly-X-Dap(Dnp)-K-K" where MeOCGly corresponds to 7-methylcoumarin-4-acetyl glycine, Dap(Dnp) corresponds to diaminoproprionyl(dinitrophenyl), and X corresponds to a sequence of natural amino acids as specified according to the standard single letter code:

1) MeOCGly- GGPGPGGDap(DNP)KK -NH2
2) MeOCGly- GGPFPGG Dap(DNP)KK -NH2
3) MeOCGly- GGPYPGG Dap(DNP)KK -NH2
4) MeOCGly- GGPNPGG Dap(DNP)KK -NH2
5) MeOCGly- GGPQPGG Dap(DNP)KK -NH2
6) MeOCGly- GGPHPGG Dap(DNP)KK -NH2
7) MeOCGly- GGPSPGG Dap(DNP)KK -NH2

8) MeOCGly- GGPDPGG Dap(DNP)KK -NH2
9) MeOCGly- GGPPPGG Dap(DNP)KK -NH2
10) MeOCGly- GGPPFGG Dap(DNP)KK -NH2
11) MeOCGly- GGPNPNPGG Dap(DNP)KK -NH2
12) MeOCGly- GGPNPGPNPGG Dap(DNP)KK -NH2
13) MeOCGly- GGPYPYPGG Dap(DNP)KK -NH2
14) MeOCGly- GGPYPGPYPGG Dap(DNP)KK -NH2
15) MeOCGly- GGNFAGG Dap(DNP)KK -NH2
16) MeOCGly- GGNFVGG Dap(DNP)KK -NH2
17) MeOCGly- GGNYAGG Dap(DNP)KK -NH2
18) MeOCGly- GGNYVGG Dap(DNP)KK -NH2
19) MeOCGly- GGQFAGG Dap(DNP)KK -NH2
20) MeOCGly- GGQFVGG Dap(DNP)KK -NH2
21) MeOCGly- GGQYAGG Dap(DNP)KK -NH2
22) MeOCGly- GGQYVGG Dap(DNP)KK -NH2

Additional specific examples of unique fluorogenic probes for detection of invasive aspergillosis include the following sequences in the format of "5(6)FAM-X-Lys(5(6)TMR-K-K" where 5(6)FAM corresponds to 5(6)-carboxyfluorescein, Lys(5(6)TMRcorresponds to Lysine(5(6)-tetramethylrhodamine), and X corresponds to a sequence of natural amino acids as specified according to the standard single letter code:

1) 5(6)FAM- GGGPGPGG Lys(5(6)TMR)KK -NH2
2) 5(6)FAM- GGGPFPGG Lys(5(6)TMR)KK -NH2
3) 5(6)FAM- GGGPQPGG Lys(5(6)TMR)KK -NH2
4) 5(6)FAM- GGGPNPGG Lys(5(6)TMR)KK -NH2
5) 5(6)FAM- GGGPYPGG Lys(5(6)TMR)KK -NH2
6) 5(6)FAM- GGGPNPNPGG Lys(5(6)TMR)KK -NH2
7) 5(6)FAM- GGGPNPGPNPGG Lys(5(6)TMR)KK -NH2

Additional specific examples of unique fluorogenic probes for detection of invasive aspergillosis include the following sequences in the format of "1,5EDANS-X-Lys(DABCYL)-K-K" where 1,5EDANS corresponds to 5-(2-Aminoethylamino)-1-naphthalenesulfonic acid, Lys(DABCYL) corresponds to Lys(4-(dimethylaminoazo)benzene-4-carboxylic acid), and X corresponds to a sequence of natural amino acids as specified according to the standard single letter code:

1) 1,5EDANS- GGPNPGG Lys(DABCYL) KK -NH2
2) 1,5EDANS- GGPYPGG Lys(DABCYL) KK -NH2
3) 1,5EDANS- GGPNPNPGG Lys(DABCYL) KK -NH2
4) 1,5EDANS- GGPNPGPNPGG Lys(DABCYL) KK -NH2

Additional specific examples of unique fluorogenic probes for detection of invasive aspergillosis include the following sequences in the format of "Abz-X-Dap(Dnp)-K-K" where Abz corresponds to o-aminobenzoic acid, Dap(Dnp) corresponds to diaminoproprionyl(dinitrophenyl), and X corresponds to a sequence of natural amino acids as specified according to the standard single letter code:

1) Abz- GGGPNPGG Dap(Dnp) KK -NH2
2) Abz- GGGPYPGG Dap(Dnp) KK -NH2
3) Abz- GGGPNPNPGG Dap(Dnp) KK -NH2
4) Abz- GGGPNPGPNPGG Dap(Dnp) KK -NH2

Additional specific examples of unique fluorogenic probes for detection of invasive aspergillosis include the following sequences in the format of "DANSYL-X-Lys(5(6)FAM-K-K" where DANSYL corresponds to 5-dimethylamino-1-naphthalenesulfonic acid, Lys(5(6)FAM corresponds to Lysine(5(6)-carboxylfluorescein, and X corresponds to a sequence of natural amino acids as specified according to the standard single letter code:

1) DANSYL- GGGPNPGG Lys(5(6)FAM)KK -NH2
2) DANSYL-GGGPYPGG Lys(5(6)FAM)KK-NH2
3) DANSYL-GGGPNPNPGG Lys(5(6)FAM)KK-NH2
4) DANSYL-GGGPNPGPNPGG Lys(5(6)FAM)KK-NH2

Example 2

Secreted Proteases of Aspergillus fumigatus (AF) as Targets for Diagnosis of Invasive Aspergillosis (IA)

Background: Innovative approaches are needed for rapid and accurate diagnosis of IA. The inventors are investigating AF secreted proteases as novel diagnostic targets. The AF genome encodes up to 100 secreted proteases, many of which are expressed in vivo during infection. The inventors hypothesize that internally quenched fluorogenic probes (IQFPs) derived from fungal protease substrates can be used to detect the enzymatic activity of AF proteases in the serum and bronchoalveolar lavage fluid (BALF) of infected patients, utilizing the unique thermotolerance of AF enzymes to distinguish them from host proteases.

Methods: The substrate specificity of AF in vitro culture supernatant was profiled with a combinatorial IQFP library in comparison with human serum to identify fungus-specific substrates. An established guinea pig model of IA was used to collect BALF during active disease for in vivo protease substrate profiling.

Results: In vitro protease substrate profiling identified approximately 75 IQFPs that were cleaved strongly by AF culture supernatant (greater than 4x fluorescence enhancement) but were not cleaved by human serum. A subset of IQFPs selected for further analysis corresponded to serine proteases that retained proteolytic activity up to 50°C. Protease substrate profiling of BALF from AF-infected guinea pigs revealed several probes that are cleaved preferentially during infection, which are promising diagnostic candidates.

Conclusions: Although IQFPs have long been used to image protease activity in vivo in oncology and inflammation, this may be the first demonstration of protease profiling for in vitro diagnosis of infection. The technology represents a promising alternative for diagnosis of IA and other blood-borne and pulmonary pathogens.

Example 3

Early Diagnosis of Invasive Aspergillosis (IA)

Methods: Guinea pig inhalation model comprises neutropenia by cortisone acetate and cyclophosphamide (days -2 and +3), $1.2*10^8$ conidia/mL nebulized AF in inhalation chamber, and serum and BALF obtained at predetermined time points; Vallor et al. Antimicrob. Ag. Chemother. 2008, 52.

Results: Proline-containing targets: PXP probes significantly diagnostic (fold ration >2.0) of infected BALF, followed by P-F/Y-X, P-S/T-X and P-X-A/V. FIGS. 1-5.

Conclusions: Cleavage of P-X-P substrates by infected guinea pig BALF at Day 7 post-infection is repeatable and highly significant; sensitivity and specificity at Day 7 are comparable to or better than existing assays (81%, 90%); and cleavage occurs primarily at P-XP and PX-P (human and fungal prolyl endopeptidases cleave at P-XP).

Example 4

Identification of Protease Substrates as Differential Diagnostic Biomarkers in Lung Fluid and Serum

Methods: Guinea pig inhalation model comprises neutropenia by cortisone acetate and cyclophosphamide (days -2 and +3), $1.2*10^8$ conidia/mL nebulized AF in inhalation chamber, and serum and BALF obtained at predetermined time points; Vallor et al. Antimicrob. Ag. Chemother. 2008, 52.

Results: The protease substrate specificities of serum and BALF from guinea pigs were determined. Differences in the proteolytic fingerprints of the two fluids were striking: serum proteases cleaved substrates containing cationic residues and proline, whereas BALF proteases cleaved substrates containing aliphatic and aromatic residues. Notably, cleavage of proline-containing substrates was virtually absent in BALF derived from healthy, uninfected guinea pigs. FIGS. 5-7.

Conclusions: Substrate specificity profiling can complement existing proteomics techniques in assessment of differences in protease specificity between complex samples. Measurements of abundance alone may not be sufficient for identification of diagnostic or therapeutic targets, because the identified proteases may be inactive or proteolytically degraded. Once proteolytic specificities of interest are identified, the responsible proteases can be identified through the use of active site-directed probes designed from the known substrate specificity. This Example represents a simple but robust method for mapping the proteolytic specificities of complex biological fluids. Substrates identified using this method may serve as sensors for diagnosis and imaging purposes, scaffolds for focused synthesis of substrates and inhibitors, probes in the design of robust assays for inhibitors, and triggers for controlled drug delivery.

Table 1. Wells enhanced in infected guinea pigs

| Plate | Well | Fold enhancement | Fold infected | Fold uninfected | FI infected | FI uninfected | Sequence (#) | Sequence (code) | Sequence |
|---|---|---|---|---|---|---|---|---|---|
| 4 | B6 | 2.13 | 2.18 | 1.02 | 325 | 188 | 324 | P-393-352 | P-A/V-N/Q |
| 4 | D6 | 2.62 | 2.85 | 1.09 | 488 | 234 | 326 | P-393-P | P-A/V-P |
| 4 | D7 | 2.37 | 2.88 | 1.22 | 498 | 213 | 334 | P-351-P | P-K/R-P |
| 4 | H7 | 2.02 | 1.64 | 0.81 | 161 | 118 | 338 | P-350-351 | P-D/E-K/R |
| 4 | D8 | 2.31 | 2.3 | 1 | 349 | 213 | 342 | P-350-P | P-D/E-P |
| 4 | G8 | 3.33 | 4.02 | 1.21 | 394 | 151 | 345 | P-352-393 | P-N/Q-A/V |
| 4 | D9 | 2.76 | 3.93 | 1.43 | 598 | 261 | 350 | P-352-P | P-N/Q-P |
| 4 | D10 | 2.21 | 2.74 | 1.24 | 474 | 223 | 358 | P-407-P | P-I/L-P |
| 4 | G11 | 2.39 | 4.28 | 1.8 | 467 | 176 | 369 | P-223-393 | P-S/T-A/V |
| 4 | D12 | 2.81 | 3.63 | 1.29 | 665 | 277 | 374 | P-223-P | P-S/T-P |
| 4 | F12 | 3.08 | 3.31 | 1.07 | 397 | 146 | 376 | P-223-354 | P-S/T-F/Y |
| 5 | A1 | 2.04 | 3.06 | 1.5 | 493 | 294 | 377 | P-354-393 | P-F/Y-A/V |
| 5 | B1 | 2.06 | 2.09 | 1.01 | 238 | 151 | 378 | P-354-351 | P-F/Y-K/R |
| 5 | D1 | 2.15 | 2.8 | 1.31 | 286 | 154 | 380 | P-354-352 | P-F/Y-N/Q |
| 5 | F1 | 4.07 | 4.71 | 1.16 | 424 | 124 | 382 | P-354-P | P-F/Y-P |

Table 2. Summary of results for screening probes against Aspergillus fumigatus infected and uninfected guinea pig broncheoaveolar lavage fluid.

| Peptide Probes | *Aspergillus fumigatus* Strain | Infected BALF | | | Uninfected BALF | | | %FE | P values | Sensitivity | Specificity |
|---|---|---|---|---|---|---|---|---|---|---|---|
| | | N | Mean Fold change | SD | N | Mean Fold Change | SD | | | | |
| PNP* | AF293 | 16 | 5.68 | 3.11 | 16 | 1.52 | 0.70 | 274.00 | 0.0001 | 0.69 | 0.97 |
| | CEA10 | 22 | 13.68 | 10.13 | 19 | 3.88 | 2.34 | 252.39 | 0.0001 | 0.95 | 0.42 |
| PYP* | AF293 | 16 | 5.42 | 3.00 | 16 | 1.79 | 1.00 | 203.00 | 0.0001 | 0.69 | 0.97 |
| | CEA10 | 21 | 14.62 | 13.89 | 17 | 3.56 | 1.95 | 310.44 | 0.0016 | 0.76 | 0.53 |
| PFP | AF293 | 22 | 6.58 | 9.44 | 23 | 2.01 | 2.45 | 227.09 | 0.0377 | 0.60 | 0.91 |
| | CEA10 | 25 | 12.44 | 11.35 | 20 | 2.92 | 1.86 | 326.24 | 0.0003 | 0.80 | 0.75 |
| PHP | AF293 | 22 | 4.47 | 4.48 | 23 | 1.40 | 0.79 | 219.69 | 0.0043 | 0.41 | 0.91 |
| | CEA10 | 25 | 10.61 | 9.51 | 20 | 3.34 | 2.27 | 217.59 | 0.0009 | 0.80 | 0.55 |
| PSP | AF293 | 20 | 12.49 | 29.69 | 22 | 3.05 | 1.30 | 309.39 | 0.1716 | 0.75 | 0.64 |
| | CEA10 | 23 | 11.00 | 8.05 | 20 | 4.54 | 3.05 | 142.55 | 0.0012 | 0.91 | 0.30 |
| PNPNP | AF293 | 20 | 13.47 | 26.98 | 19 | 2.96 | 1.41 | 355.02 | 0.0979 | 0.75 | 0.68 |
| | CEA10 | 23 | 8.72 | 5.54 | 20 | 4.13 | 2.39 | 111.20 | 0.0010 | 0.83 | 0.40 |

Table 2 Legend. Variable region (Xaa-Yaa-Zaa) of the top 6 peptide probes are all listed in single amino acid code; AF293 and CEA10 are the two strains of *Aspergillus fumigatus* infected guinea pig BALF samples screened in this study.

N stands for number of guinea pig BALF samples screened against the given peptide probe. Guinea pig infected and uninfected samples with very low background (t=0 hour) and end point values (t=6 hour) (i.e., no greater than 100 units difference between t=0 hour and t=6 hour) were not selected for the statistical calculations.

Mean fold change values is the average of fluorescence fold change calculated for each guinea pig sample screened against respective peptide probe. Fluorescence fold change values are calculated according to the formula:

$$\text{Fold Change}=\text{Fluorescence measured at time t=6 hour/Fluorescence measured at time t=0 hour.}$$

* Fold change values for these peptide probes were calculated at time t=3 hour as the end point value. SD corresponds to standard deviation calculated for the given probe screened against the respective BALF sample.

Percentage fluorescence efficiency (% FE) is calculated for each probe as follows, % FE=100 * [(Fold $\text{change}_{\text{Infected BALF}}$ -Fold $\text{change}_{\text{Uninfected BALF}}$) / Fold $\text{change}_{\text{uninfected BALF}}$] Fold change values are all the mean fold change values.

P values are calculated with two tailed student t-test with two sample unequal variance for each peptide probe screened against respective infected and uninfected BALF samples.

Sensitivity and Specificity of the assay for each peptide probe was calculated as shown below,

$$\text{Sensitivity}=\text{True positives/(True positives+False negatives)}$$

$$\text{Specificity}=\text{True negatives/(True negatives +False positives)}$$

True positive=Fold change 3 or greater in infected guinea pig sample

True negative=Fold change less than 3 in uninfected guinea pig sample

False positive=Fold change 3 or greater in uninfected guinea pig sample

False negative=Fold change less than 3 in infected guinea pig sample.

The descriptions of particular embodiments and examples are offered by way of illustration and not by way of limitation.

## Claims

1. A method of detecting prolyl endopeptidase (PE) activity in a lung sample, comprising:

    (a) contacting the sample with a probe comprising a -P-X- or -P-X-P- prolyl endopeptidase recognition site, wherein P is a prolyl bioisostere, X is (i) N, F, Y, S, H or G, or (ii) a prolyl bioisostere flanked on each side by N, F, Y, S, H or G, or (iii) aminoluciferin, and
    "-" is an amide bond, under conditions wherein prolyl endopeptidase activity of the sample specifically hydrolyzes an amide bond of the recognition site to generate an optical signal; and
    (b) detecting the signal.

2. The method of claim 1 wherein the probe comprises a -P-X-P-X- prolyl endopeptidase recognition site.

3. The method of claim 1 wherein the prolyl bioisostere is selected from substituted or unsubstituted proline, homoproline, hydroxyproline, dehydroproline, aminoproline, 5,6-benzohomoproline, alkylproline, N-methylamino acid, and decahydroisoquinoline carboxylate.

4. The method of claim 1 wherein the probe

    (i) further comprises a hydrophilic moiety and the probe is water soluble.
    (ii) is internally quenched.

5. The method of claim 1 wherein one end of the recognition site is operably-linked to a first chromophore, and the other end of the recognition site is operably-linked to a second chromophore that quenches the first chromophore.

6. The method of claim 1 wherein one end of the recognition site is operably-linked to a fluorescence resonance energy transfer (FRET) donor, and the other end of the recognition site is operably-linked to a FRET acceptor, and the donor and acceptor are independently operably-linked through a linker that is glycine, serine, a peptide of serine and/or glycine, a 8-amino-3,6-dioxaoctanoic acid or 11-amino-3,6,9-trioxaundecanoic acid, or a linear aliphatic alpha-amino acid.

7. The method of claim 1 wherein proteolysis of the probe produces a substrate, and the sample is further contacted with an enzyme that reacts with the substrate to produce the signal.

8. The method of claim 1 wherein the sample is bronchoalveolar lavage fluid of a patient having a lung disease.

9. The method of claim 1 wherein the lung has a disease that is

(i) a lung infection that is a fungal infection, a bacterial infection, a parasitic infection, or a viral infection;
(ii) a non-infectious inflammatory disease; or
(iii) invasive aspergillosis.

10. The method of claim 1 wherein the contacting and detecting steps are repeated with different PE probes with different lung samples, wherein the method determines one or more differences in PE activity between the samples.

## Patentansprüche

1. Ein Verfahren zum Nachweis von Prolyl Endopeptidase (PE)-Aktivität in einer Lungenprobe, umfassend:

(a) Kontaktieren der Probe mit einer Sonde, die eine -P-X- oder -P-X-P- Prolyl-Endopeptidase Erkennungsstelle umfasst, wobei P ein Prolyl-Bioisoster ist, X (i) N, F, Y, S, H oder G ist oder (ii) ein Prolyl-Bioisoster ist, das an jeder Seite von N, F, Y, S, H oder G flankiert wird, oder (iii) Aminoluziferin, und
"-" eine Amid-Bindung ist, unter Bedingungen, wobei die Prolyl Endopeptidase-Aktivität der Probe spezifisch eine Amid-Bindung der Erkennungsstelle hydrolysiert, um ein optisches Signal zu erzeugen; und
(b) Nachweisen des Signals.

2. Das Verfahren gemäß Anspruch 1, wobei die Sonde eine -P-X-P-X- Prolyl Endopeptidase Erkennungsstelle umfasst.

3. Das Verfahren gemäß Anspruch 1, wobei das Prolyl-Bioisoster aus substituiertem oder unsubstituiertem Prolin, Homoprolin, Hydroxyprolin, Dehydroprolin, Aminoprolin, 5,6-Benzohomoprolin, Alkylprolin, N-Methylaminosäure und Decahydroisochinolincarboxylat ausgewählt ist.

4. Das Verfahren gemäß Anspruch 1, wobei die Sonde

(i) ferner eine hydrophile funktionelle Gruppe umfasst und die Sonde wasserlöslich ist,
(ii) intern gequencht ist.

5. Das Verfahren gemäß Anspruch 1, wobei ein Ende der Erkennungsstelle mit einem ersten Chromophor operativ verknüpft ist und das andere Ende der Erkennungsstelle mit einem zweiten Chromophor, das das erste Chromophor quencht, operativ verknüpft ist.

6. Das Verfahren gemäß Anspruch 1, wobei ein Ende der Erkennungsstelle mit einem Fluoreszenz-Resonanz-Energietransfer (FRET) Donor operativ verknüpft ist und das andere Ende der Erkennungsstelle mit einem FRET Akzeptor operativ verknüpft ist und der Donor und der Akzeptor unabhängig voneinander durch einen Linker, der Glycin, Serin, ein Peptid aus Serin und/oder Glycin, eine 8-Amino-3,6-dioxaoctansäure oder eine 11-Amino-3,6,9-trioxaundecansäure oder eine lineare aliphatische alpha-Aminosäure ist, operativ verknüpft sind.

7. Das Verfahren gemäß Anspruch 1, wobei die Proteolyse der Sonde ein Substrat erzeugt und die Probe ferner mit einem Enzym kontaktiert wird, das mit dem Substrat reagiert, um das Signal zu erzeugen.

8. Das Verfahren gemäß Anspruch 1, wobei die Probe eine bronchoalveoläre Spülflüssigkeit eines Patienten, der eine Lungenerkrankung hat, ist.

9. Das Verfahren gemäß Anspruch 1, wobei die Lunge eine Erkrankung hat, die

   (i) eine Lungeninfektion ist, die eine Pilzinfektion, eine bakterielle Infektion, eine parasitäre Infektion oder eine virale Infektion ist;
   (ii) eine nicht-infektiöse Entzündungserkrankung; oder
   (iii) invasive Aspergillose
   ist.

10. Das Verfahren gemäß Anspruch 1, wobei die Kontaktierungs- und Nachweisschritte mit unterschiedlichen PE-Sonden mit unterschiedlichen Lungenproben wiederholt werden, wobei das Verfahren einen oder mehrere Unterschiede der PE-Aktivität zwischen den Proben bestimmt.

## Revendications

1. Procédé de détection d'activité de prolyle endopeptidase (PE) dans un échantillon de poumon, comprenant :

   (a) la mise en contact de l'échantillon avec une sonde comprenant un site de reconnaissance de la prolyle endopeptidase de type -P-X- ou -P-X-P-, dans lequel P est un bio-isostère de prolyle, X est (i) N, F, Y, S, H ou G, ou (ii) un bio-isostère de prolyle flanqué sur chaque côté par N, F, Y, S, H ou G, ou (iii) une aminoluciférine, et « - » est une liaison amide, dans des conditions dans lesquelles l'activité de la prolyle endopeptidase de l'échantillon hydrolyse de manière spécifique une liaison amide du site de reconnaissance pour générer un signal optique ; et
   (b) la détection du signal.

2. Procédé selon la revendication 1, dans lequel la sonde comprend un site de reconnaissance de la prolyle endopeptidase -P-X-P-X-.

3. Procédé selon la revendication 1, dans lequel le bio-isostère de prolyle est choisi parmi la proline substituée ou non substituée, l'homoproline, l'hydroxyproline, la déshydroproline, l'aminoproline, la 5,6-benzohomoproline, l'alkylproline, l'acide N-méthylamino, et le carboxylate de décahydroisoquinoléine.

4. Procédé selon la revendication 1, dans lequel la sonde

   (i) comprend en outre un fragment hydrophile et la sonde est hydrosoluble,
   (ii) est trempée de manière interne.

5. Procédé selon la revendication 1, dans lequel une extrémité du site de reconnaissance est liée de manière opérationnelle à un premier chromophore, et l'autre extrémité du site de reconnaissance est liée de manière opérationnelle à un second chromophore qui trempe le premier chromophore.

6. Procédé selon la revendication 1, dans lequel une extrémité du site de reconnaissance est liée de manière opérationnelle à un donneur de transfert d'énergie de fluorescence par résonance (FRET), et l'autre extrémité du site de reconnaissance est liée de manière opérationnelle à un accepteur de FRET, et le donneur et l'accepteur sont indépendamment liés de manière opérationnelle par un lieur qui est une glycine, une sérine, un peptide de sérine et/ou de glycine, un acide 8-amino-3,6-dioxaoctanoïque ou un acide 11-amino-3,6,9-trioxaundécanoïque, ou un acide alpha-amino aliphatique linéaire.

7. Procédé selon la revendication 1, dans lequel la protéolyse de la sonde produit un substrat, et l'échantillon est en outre mis en contact avec une enzyme qui réagit avec le substrat pour produire le signal.

8. Procédé selon la revendication 1, dans lequel l'échantillon est un liquide de lavage broncho-alvéolaire d'un patient atteint d'une maladie pulmonaire.

9. Procédé selon la revendication 1, dans lequel le poumon a une maladie qui est

(i) une infection pulmonaire qui est une infection fongique, une infection bactérienne, une infection parasitaire, ou une infection virale ;
(ii) une maladie inflammatoire non infectieuse ; ou
(iii) l'aspergillose invasive.

**10.** Procédé selon la revendication 1, dans lequel les étapes de mise en contact et de détection sont répétées avec différentes sondes de PE et avec différents échantillons de poumon, dans lequel le procédé détermine une ou plusieurs différences dans l'activité de la PE entre les échantillons.

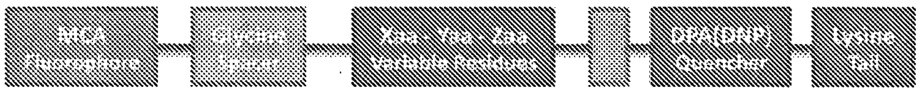
MCA
Fluorophore
Glycine
Spacer
Xaa - Yaa - Zaa
Variable Residues
DPA(DNP)
Quencher
Lysine
Tail

Fig. 1

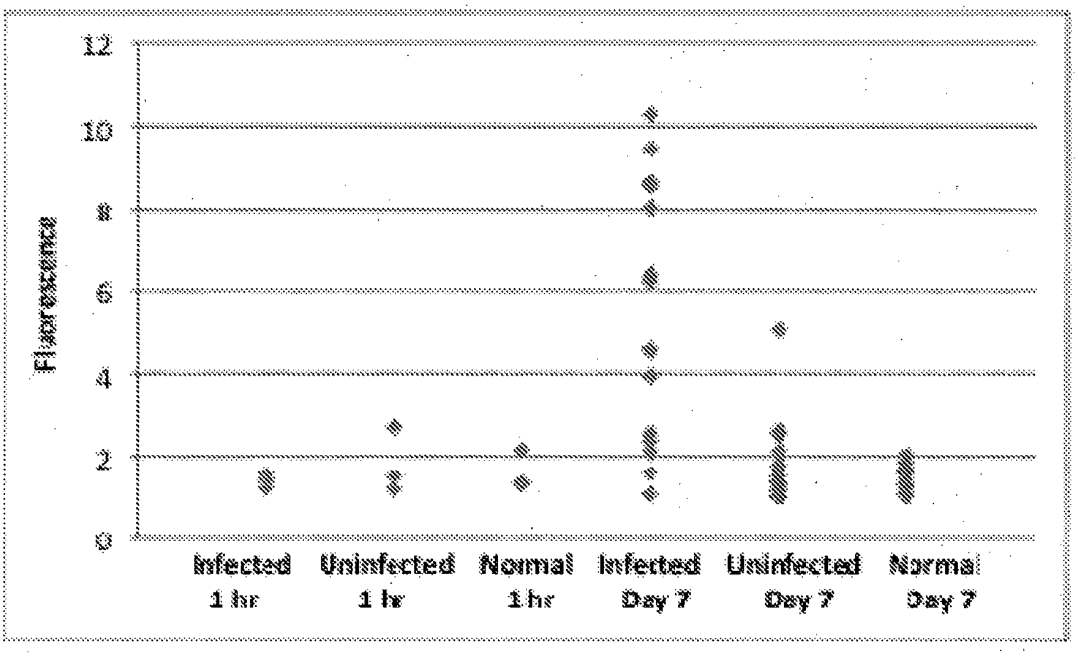
Fluorescence
12
10
8
6
4
2
0
Infected 1 hr
Uninfected 1 hr
Normal 1 hr
Infected Day 7
Uninfected Day 7
Normal Day 7

Fig. 2

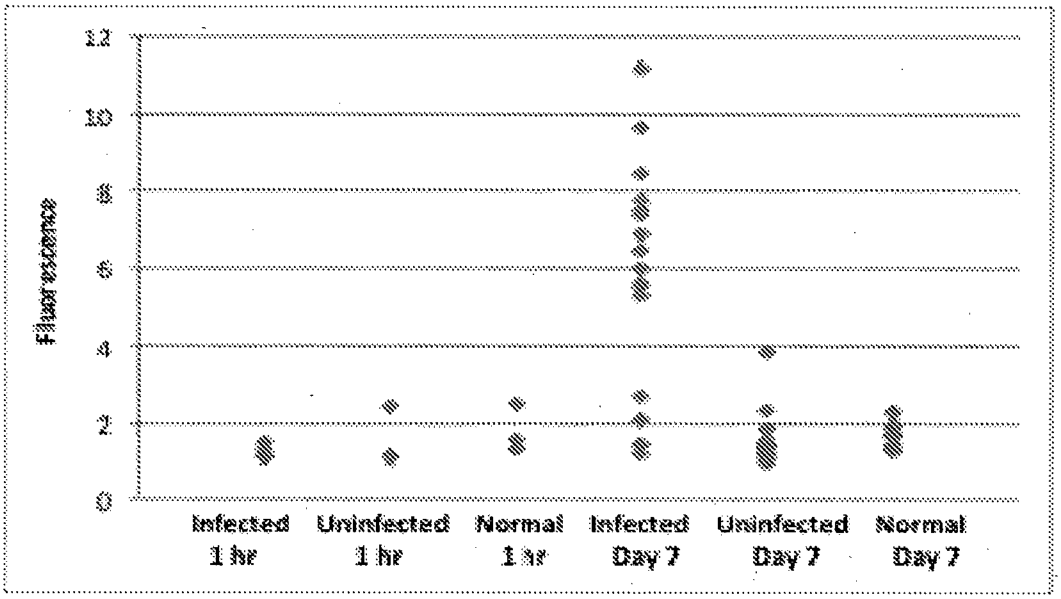
12
10
8
6
4
2
0
Fluorescence
Infected 1 hr
Uninfected 1 hr
Normal 1 hr
Infected Day 7
Uninfected Day 7
Normal Day 7

Fig. 3

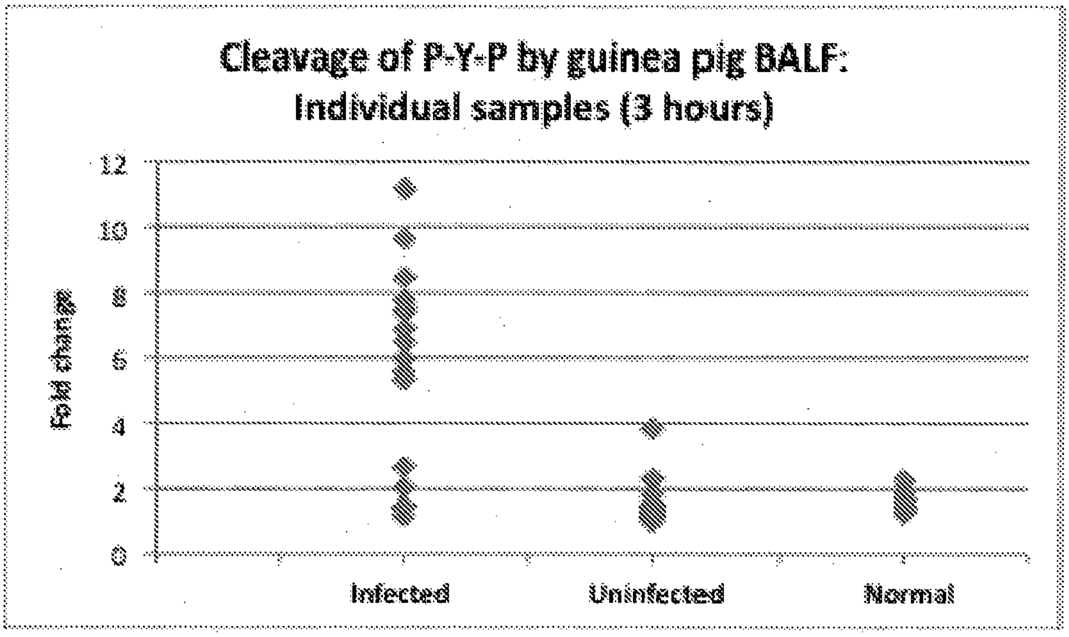
Cleavage of P-Y-P by guinea pig BALF:
Individual samples (3 hours)
12
10
8
6
4
2
0
Fold change
Infected
Uninfected
Normal

Fig. 4

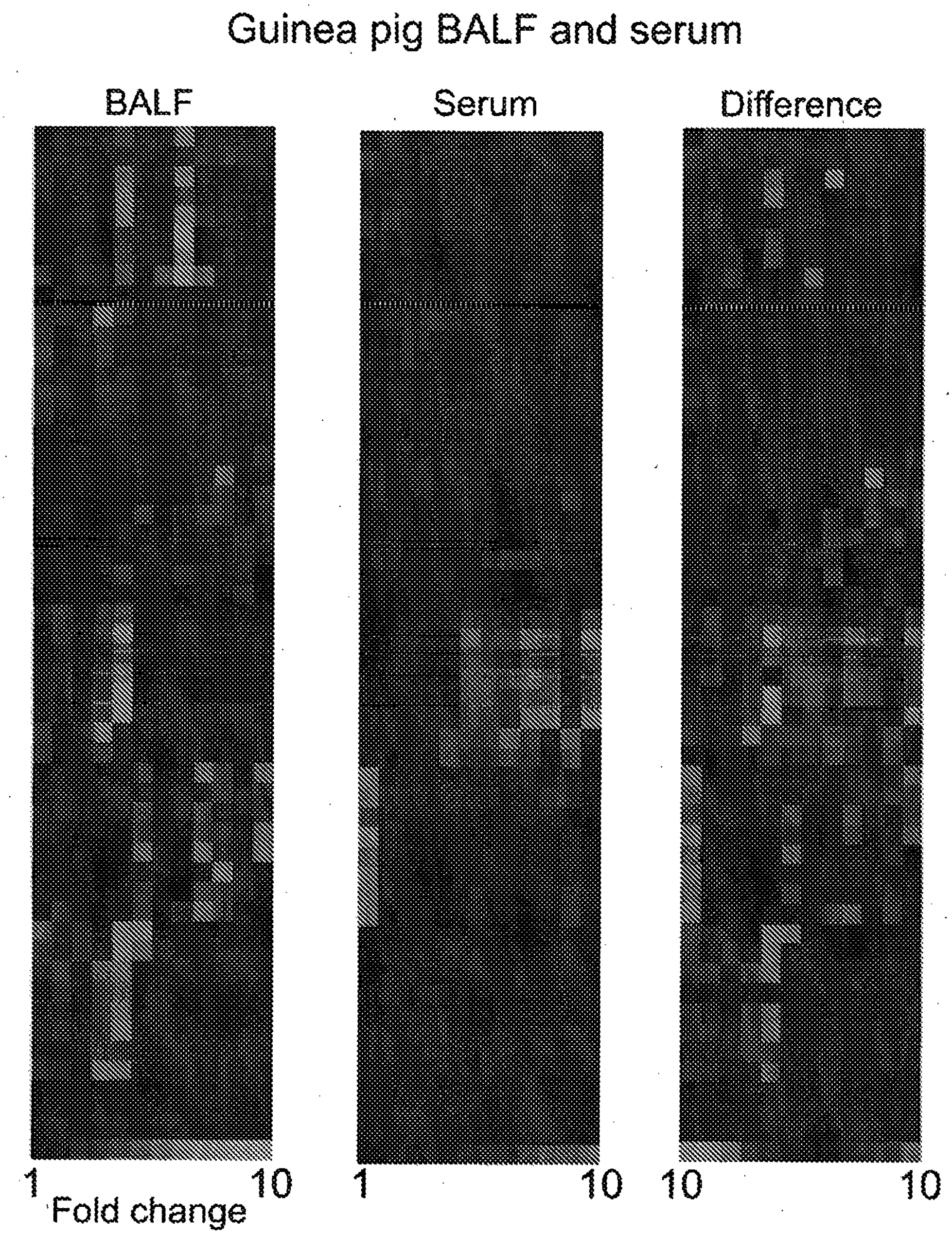
Guinea pig BALF and serum
BALF
Serum
Difference
1
10
Fold change
1
10
10
10

Fig. 5

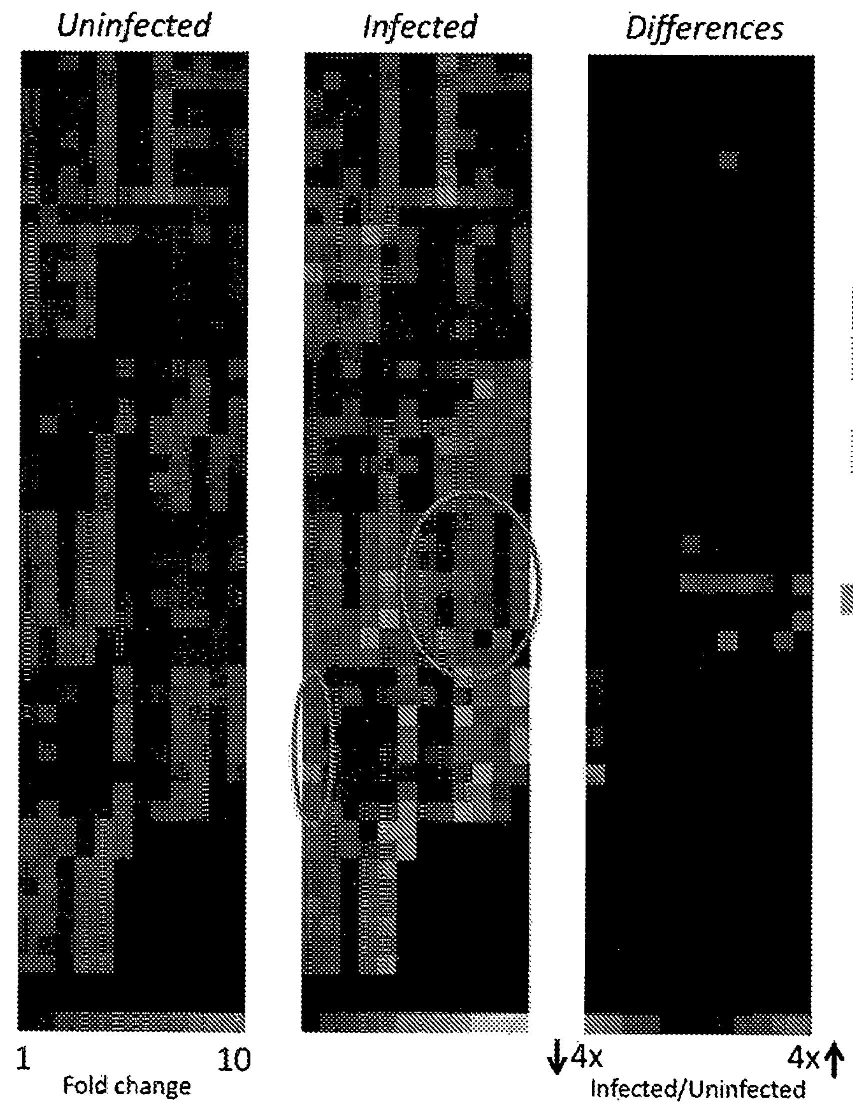


*In vivo* diagnostic candidates:

| Fold change | Number of wells | Percent |
|---|---|---|
| 2 to 3 | 12 | 2.3 |
| 3 to 4 | 2 | 0.4 |
| 4 or more | 1 | 0.2 |

$$\text{Fold change} = \frac{\text{Fluorescence}_{6h}}{\text{Fluorescence}_{0h}}$$

$$\text{Differences} = \frac{\text{Fold}_{\text{Infected}}}{\text{Fold}_{\text{Uninfected}}}$$

Fig. 6.

A Amino acid distribution of sequences cleaved by BALF:

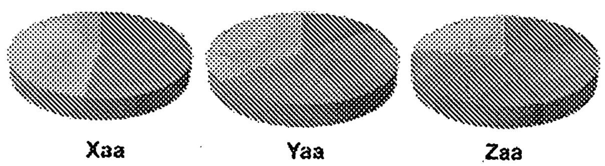


P S/T D/E K/R N/Q F/Y A/V I/L

B Amino acid distribution of sequences cleaved by serum:

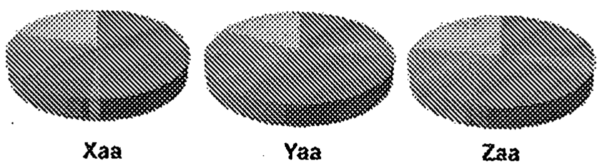


P S/T D/E K/R N/Q F/Y A/V I/L

C Motifs cleaved by BALF:

| Xaa | Yaa | Zaa | Fold Change | Linear Slope | Nonlinear Rate |
|---|---|---|---|---|---|
| I/L | F/Y | --- | 5.75 | 83.3 | 0.123 |
| F/Y | F/Y | --- | 5.28 | 72.4 | 0.214 |
| F/Y | S/T | --- | 5.05 | 82.7 | 0.131 |
| F/Y | --- | S/T | 4.85 | 97.1 | 0.121 |
| F/Y | --- | A/V | 4.61 | 73.4 | 0.166 |
| F/Y | D/E | --- | 4.55 | 76.5 | 0.097 |
| --- | F/Y | D/E | 4.52 | 72.0 | 0.118 |
| --- | S/T | F/Y | 4.50 | 63.6 | 0.066 |
| F/Y | --- | N/Q | 4.40 | 58.9 | 0.178 |
| I/L | --- | F/Y | 4.29 | 59.4 | 0.095 |

D Motifs cleaved by serum:

| Xaa | Yaa | Zaa | Fold Change | Linear Slope | Nonlinear Rate |
|---|---|---|---|---|---|
| P | F/Y | --- | 10.63 | 158 | 0.308 |
| P | --- | N/Q | 8.89 | 155 | 0.392 |
| P | --- | P | 8.40 | 180 | 0.590 |
| P | S/T | --- | 8.29 | 157 | 0.430 |
| P | --- | A/V | 7.95 | 155 | 0.375 |
| P | N/Q | --- | 7.77 | 146 | 0.448 |
| P | --- | S/T | 7.61 | 132 | 0.256 |
| P | A/V | --- | 6.84 | 140 | 0.319 |
| P | I/L | --- | 6.65 | 134 | 0.350 |
| P | --- | I/L | 6.36 | 118 | 0.208 |

E Distribution of BALF protease cleavage rates:

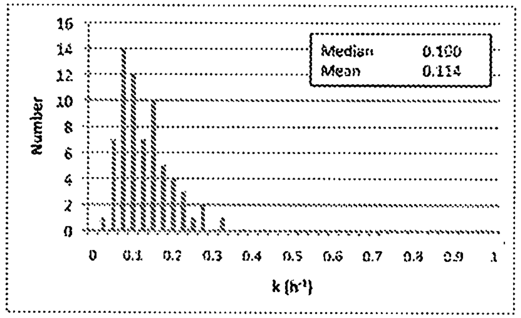


F Distribution of serum protease cleavage rates:

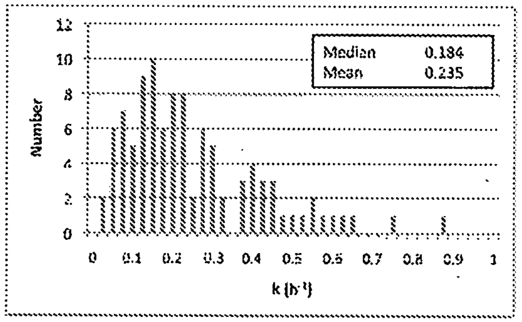


Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007087131 A2


### Non-patent literature cited in the description


- **ORLOWSKI. M. et al.** *Journal of laboratory and clinical medicine,* 1981, vol. 97 (4), 467-476
- **QUINTO, B.M.R. et al.** *International Journal of Biochemistry and Cell Biology,* 2000, vol. 32 (11-12), 1161-1172
- **FULOP VILMOS et al.** *Journal of Biological Chemistry,* 2001, vol. 276 (2), 1262-1266
- **WATSON et al.** *BioTechiques,* August 2011, vol. 51, 95-104
- **WATSON et al.** *PLoS ONE,* June 2011, vol. 6 (6), e21001
- **SAMPOGNARO et al.** *Bioorg Med Chem Lett.,* 01 September 2010, vol. 20 (17), 5027-30
- **NILSSON et al.** *J Comb Chem.,* November 2001, vol. 3 (6), 546-53
- **THORSTENSSON.** *Linköping Studies in Science and Technology, Dissertations No. 990,* 2005
- **SHUMAN et al.** *J Org Chem,* 1990, vol. 55, 738-41
- **VALLOR et al.** *Antimicrob. Ag. Chemother.,* 2008, 52